# EUROPEAN PATENT SPECIFICATION

(11) **EP 2 124 919 B1**
(45) Date of publication and mention of the grant of the patent: **16.05.2012**
(21) Application number: 08725690.5
(22) Date of filing: 15.02.2008
(51) Int. Cl.: A61K 31/513, A61P 19/00

(54) **5,5'-(sulfonyldiemethylene)diuracil for modulating bone formation and mineralization**
5,5'-(sulfonyldiemethylene)diuracil zur Modulation von Knochenbildung und -Mineralisierung
5,5'-(sulfonyldiemethylene)diuracil pour moduler une formation et une minéralisation osseuses

(30) Priority: 16.02.2007 US 901753 P
(43) Date of publication of application: 02.12.2009
(73) Proprietor: President and Fellows of Harvard College, Cambridge, MA 02138 (US)
(72) Inventor: GLIMCHER, Laurie, H., West Newton, MA 02165 (US)
(74) Representative: Nash, David Allan
(86) International application number: PCT/US2008/002082
(87) International publication number: WO 2008/103314

(56) References cited:
- WO-A-2005/042726
- WO-A-2006/113559
- JONES D C ET AL: "Regulation of adult bone mass by the zinc finger adapter protein Schnurri-3" SCIENCE, AMERICAN ASSOCIATION FOR THE ADVANCEMENT OF SCIENCE, US, WASHINGTON, DC, vol. 312, no. 5777, 1 May 2006 (2006-05-01), pages 1223-1227, XP002403273 ISSN: 0036-8075
- HOFSTAETTER J G ET AL: "High bone mass and increased degree of mineralization in Schnurri-3 null mice" BONE (NEW YORK), vol. 39, no. 5, November 2006 (2006-11), page S18, XP002517727 & INTERNATIONAL CONFERENCE ON PROGRESS IN BONE AND MINERAL RESEARCH 2006; VIENNA, AUSTRIA; NOVEMBER 16 -18, 2006 ISSN: 8756-3282

## Description

### Background of the Invention:

Transcription factors are a group of molecules within the cell that function to connect the pathways from extracellular signals to intracellular responses. Immediately after an environmental stimulus, these proteins which reside predominantly in the cytosol are translocated to the nucleus where they bind to specific DNA sequences in the promoter elements of target genes and activate the transcription of these target genes. One family of transcription factors, the ZAS (zinc finger-acidic domain structures) DNA binding protein family is involved in the regulation of gene transcription, DNA recombination, and signal transduction (Mak, C.H., et al. 1998. Immunogenetics 48: 32-39).

Zinc finger proteins are identified by the presence of highly conserved Cys2His2 zinc fingers (Mak, C.H., et al. 1998. Immunogenetics 48: 32-39). The zinc fingers are an integral part of the DNA binding structure called the ZAS domain. The ZAS domain is comprised of a pair of zinc fingers, a glutamic acid/aspartic acid-rich acidic sequence and a serine/threonine rich sequence (Mak, C.H., et al. 1998. Immunogenetics 48: 32-39). The ZAS domains have been shown to interact with the kB like cis-acting regulatory elements found in the promoter or enhancer regions of genes. The ZAS proteins recognize nuclear factor kB binding sites which are present in the enhancer sequences of many genes, especially those involved in immune responses (Bachmeyer, et al. 1999. Nuc. Acid Res. 27, 643-648). The ZAS DNA binding proteins have been shown to be transcription regulators of these target genes (Bachmeyer, et al. 1999. Nuc. Acid Res. 27, 643-648; Wu et al. 1998. Science 281, 998-1001).

The zinc finger transcription factor schnurri3 or Shn3, also known as Kappa Recognition Component or "KRC", and human immunodeficiency virus type I enhancer-binding protein 3 (HIVEP3)) is a member of the ZAS DNA binding family of proteins (Bachmeyer, et al. 1999. Nuc. Acid Res. 27, 643-648; Wu et al. 1998. Science 281, 998-1001). The Shn3 gene was identified as a DNA binding protein for the heptameric consensus signal sequences involved in somatic V(D)J recombination of the immune receptor genes (Mak, C. H., et al. 1994. Nuc. Acid Res. 22: 383-390). Shn3 is a substrate for epidermal growth factor receptor kinase and p34cdc2 kinase in vitro (Bachmeyer, et al. 1999. Nuc. Acid Res 27, 643-648).

In *Drosophila,* Schnurri (Shn) plays an important role during embryogenesis in the regulation of genes downstream of decapentaplegic (Dpp), a member of the TGF-β superfamily (Arora, K., et al. (1995). Cell 81, 781-790). Ligation of Dpp to its receptors initiates a signal cascade that results in Med, the Drosophila Co-Smad homologue, partnering with Mad, the Drosophila R-Smad homologue (Dai, H., et al. (2000). Dev Biol 227, 373-387). The Mad/Med complex translocates to the nucleus where it interacts with Shn. It has been demonstrated that Shn recruits the necessary transcriptional co-repressors to the Mad/Med complex bound to the regulatory region of Brinker (Brk). Since Brk is a global repressor of Dpp-mediated gene expression, Shn-induced repression of Brk expression thus promotes Dpp's ability to induce expression of target genes (Arora, K., et al. (1995). Cell 81, 781-790; Dai, H., et al. (2000). Dev Biol 227, 373-387; Marty, T., et al. (2000). Nat Cell Biol 2, 745-749).

Although a number of studies have demonstrated that Shn3 regulates the activities of other important transcription proteins, including NF-κB and AP-1, no role for the mammalian Shn genes in TGF-β signaling has yet to be identified (Hong, J. W., et al. (2003). Proc Natl Acad Sci U S A 100, 12301-12306; Oukka, M., et al. (2004). J Exp Med 199, 15-24; Oukka, M., et al. (2002). Mol Cell 9, 121-131). Furthermore, the *in vivo* role(s) of Shn3 remain largely unknown.

Bone is a dynamic tissue whose matrix components are continuously being remodeled to preserve the structural integrity of the skeleton. Bone remodeling is a cyclical process where under normal physiological conditions, bone formation occurs only at sites where bone resorption has previously taken place. Homeostatic remodeling of the skeleton is mediated primarily, if not exclusively, by the osteoclast and the osteoblast (Erlebacher, A., et al. (1995). Cell 80, 371-378). Osteoclasts are giant multinucleated cells of hematopoietic origin that are responsible for bone resorption. Osteoblasts, which originate from mesenchymal stem cells, synthesize the matrix constituents on bone forming surfaces. Proliferation, differentiation and bone remodeling activities of these cells involve a complex temporal network of growth factors, signaling proteins, and transcription factors (Karsenty, G., and Wagner, E. F. (2002). Dev Cell 2, 389-406). Dysregulation of any one component may disrupt the remodeling process and contribute to the pathogenesis of certain skeletal disorders, such as osteoporosis and Paget's disease. Rare single gene disorders resulting in elevated bone mass due to osteoclast defects, collectively termed osteopetrosis, have been identified. Rarer are single gene disorders, exemplified by Camerati-Engelman syndrome, collectively termed osteosclerosis, in which elevated bone mass is due to intrinsically-elevated osteoblast activity.

The transcription factor Runx2 is the principal regulator of osteoblast differentiation during embryonic development. It interacts with a number of nuclear transcription factors, coactivators, and adaptor proteins that interpret extracellular signals to ensure homeostatic osteoblast development and activity (Lian, J. B., et al. (2004). Crit Rev Eukaryot Gene Expr 14, 1-41; Stein, G. S., et al. (2004). Oncogene 23, 4315-4329). Mutations in Runx2 cause the human autosomal dominant disease cleidoranial dysplasia (Lee, B., et al. (1997). Nat Genet 16, 307-310; Mundlos, S., et al. (1997). Cell 89, 773-779; Otto, F., et al. (1997). Cell 89, 765-771). Runx2^{-/-} mice exhibit a complete lack of both intramembranous and endochondral ossification, which results in an unmineralized skeleton (Komori, T., et al. (1997). Cell 89, 755-764; Otto, F., et al. (1997). Cell 89, 765-771). In contrast to the significant progress in understanding the molecular mechanisms responsible for osteoblast differentiation during embryonic development, only a small number of genes are known to regulate postnatal osteoblast function (Yoshida, Y., et al. (2000). Cell 103, 1085-1097; Kim, S., et al. (2003). Genes Dev 17, 1979-1991). LRP5, a Wnt coreceptor, is important in the regulation of bone mass in adult humans and rodents (Johnson, M. L., et al. (2004). J Bone Miner Res 19, 1749-1757). Runx2, in addition to its central role in osteoblast differentiation, also regulates mature osteoblast activity in adult mice (Ducy, P., et al. (1999). Genes Dev 13, 1025-1036) in part through its induction of ATF4, another protein demonstrated to be important in postnatal bone formation (Yang, X., et al. (2004). Cell 117, 387-398). TGFβ has a complex function in bone homeostasis mediated in part through the activity of the SMAD3 E3 ligase, Smurf1.

### Summary of the Invention:

The present invention provides an Shn3 modulating compound for use in a method of increasing base formation and mineralisation, wherein said compound in 5,5'-(sulfonyldimethylene)uracil.

### Detailed Description of the Invention:

The present invention is based, at least in part, on the discovery of small molecules which modulate bone formation and mineralization by interacting with Shn3, Runx2, SMAD3, and/or WWP1. It has been found that TGF-β signaling in osteoblasts promotes the formation of a multimeric complex between Shn3, Runx2, Smad3, and the E3 ubiquitin ligase, WWP1, which inhibits Runx2 function due to the ability of WWP1 to promote Runx2 polyubiquitination and proteasome-dependent degradation. Shn3 is an integral and required component of this complex, since its absence in osteoblasts results in elevated levels of Runx2 protein, enhanced Runx2 transcriptional activity, elevated transcription of Runx2 target genes, profoundly increased bone formation *in vivo,* as well as defective osteoclastogenesis *in vivo.* It was also discovered previously that Shn3 and WWP1 also form a complex with RSK2 which promotes RSK2 phosphorylation and inhibits RSK2 function due to the ability of WWP1 to promote RSK2 ubiquitination.

The *Schnurri-3* (Shn3), referred to interchangeably herein as KRC protein (for κB binding and putative recognition component of the V(D)J Rss) is a DNA binding protein comprised of 2282 amino acids. Shn3 has been found to be present in T cells, B cells, and macrophages. Shn3 is a member of a family of zinc finger proteins that bind to the kB motif (Bachmeyer, C, et al., 1999. Nuc. Acids. Res. 27(2):643-648). Zinc finger proteins are divided into three classes represented by KRC and the two MHC Class I gene enhancer binding proteins, MBP1 and MBP2 (Bachmeyer, C, et al., 1999. Nuc. Acids. Res. 27(2):643-648).

### 1. Definitions

The term "Shn3" or "schnurri 3", used interchangeably with "KRC." The aminoacid and nucleotide sequence of Shn3 is given in PCT/US2006/014295, incorporated herein by reference.

The language "Shn3 family polypeptide" includes proteins or nucleic acid molecules having a Shn3 structural domain or motif and having sufficient amino acid or nucleotide sequence identity with a Shn3 molecule as defined herein. Such family members can be naturally or non-naturally occurring and can be from the same or different species. For example, a family can contain a first protein of human origin, as well as other, distinct proteins of human origin or, alternatively, can contain homologues of non-human origin. Preferred members of a family may also have common functional characteristics. Preferred Shn3 polypeptides comprise one or more of the following Shn3 characteristics: a pair of Cys2-His2 zinc fingers followed by a Glu- and Asp-rich acidic domain and five copies of the ser/Thr-Pro-X-Arg/Lys sequence thought to bind DNA.

The term "Shn3 activity," "Shn3 biological activity" or "activity of a Shn3 polypeptide" includes the ability to modulate an activity regulated by Shn3, a Shn3 family polypeptide, such as for example Shn3 tr, or a signal transduction pathway involving Shn3. For example, in one embodiment a Shn3 biological activity includes modulation of an immune response. In another embodiment, Shn3 modulates bone formation and mineralization. Exemplary Shn3 activities include *e.g*., modulating: immune cell activation and/or proliferation (such as by modulating cytokine gene expression), cell survival (*e.g*., by modulating apoptosis), signal transduction via a signaling pathway (*e.g*., an NFkB signaling pathway, a JNK signaling pathway, and/or a TGFγ signaling pathway), actin polymerization, ubiquitination of AP-1, ubiquitination of TRAF, degradation of c-Jun, degradation of c-Fos, degradation of SMAD, degradation of GATA3, GATA3 expression, modulation of Th2 cell differentiation, modulation of Th2 cytokine production, IgA production, modulation of GLα transcription, modulation of bone growth, modulation of bone mineralization, modulation of osteoclastogenesis, modulation of osteoblast versus osteoclast activity, *e.g*., in bone formation and/or remodeling of bone, modulation of osteocalcin gene transcription, degradation of Runx2, *e.g*., modulation of Runx2 protein levels, ubiquitination of Runx2, modulation of the expression of RSK2, degradation of RSK2, *e.g*., modulation of RSK2 protein levels, ubiquitination of RSK2, modulation of the phosphorylation of RSK2, modulation of the expression of BSP, ColI(α)1, OCN, Osterix, RANKL, and ATF4, modulation of ATF4 protein levels, and/or modulation of the phosphorylation of ATF4.

The various forms of the term "modulate" include stimulation (*e.g*., increasing or upregulating a particular response or activity) and inhibition (*e.g*., decreasing or downregulating a particular response or activity).

As described above, Shn3 modulates bone formation and mineralization through a complex interaction of molecules which are downstream of TGF-β signaling. In one embodiment, the Shn3 activity is a direct activity, such as an association with a Shn3-target molecule or binding partner. As used herein, a "target molecule", "binding partner" or "Shn3 binding partner" is a molecule with which a Shn3 protein binds or interacts in nature, such that Shn3 mediated function is achieved.

The term "TRAF" refers to TNF Receptor Associated Factor (See *e.g*., Wajant et al, 1999, Cytokine Growth Factor Rev 10:15-26). The "TRAF" family includes a family of cytoplasmic adapter proteins that mediate signal transduction from many members of the TNF-receptor superfamily and the interleukin-1 receptor (see *e.g*., Arch, R.H. et al., 1998, Genes Dev. 12:2821-2830). The term "TRAF C domain" refers to the highly conserved sequence motif found in TRAF family members.

The term "bone formation and mineralization" includes the cellular activity of osteoblasts to synthesize the collagenous precursors of bone extracellular matrix, regulate mineralization of the matrix to form bone, as well as their function in bone remodeling and reformation, *e.g*., bone mass is maintained by a balance between the activity of osteoblasts that form bone and the osteoclasts that break it down. The mineralization of bone occurs by deposition of carbonated hydroxyapetite crystals in an extracellular matrix consisting of type I collagen and a variety of non-collagenous proteins.

The term "osteoblast" includes bone-forming cells that are derived from mesenchymal osteoprognitor cells and forms an osseous matrix in which it becomes enclosed as an osteocyte. A mature osteoblast is one capable of forming bone extracellular matrix *in vivo* , and can be identified *in vitro* by its capacity to form mineralized nodules which reflect the generation of extracellular matrix. An immature osteoblast is not capable of forming mineralized nodules *in vitro.*

The term "osteoclast" includes large multinucleated cells with abundant acidophilic cytoplasms, functioning in the absorption and removal of osseous tissue. Osteoclasts become highly active in the presence of parathyroid hormone, causing increased bone resorption and release of bone salts (phosphorus and, especially, calcium) into the extracellular fluid.

The term "osteocalcin", also called bone Gla protein, includes a vitamin K-dependent, calcium-binding bone protein, the most abundant noncollagen protein in bone. Osteocalcin is specifically expressed in differentiated osteoblasts and odontoblasts. The TGF-β-mediated decrease of osteocalcin has been shown to occur at the mRNA level and does not require new protein synthesis. Transcription from the osteocalcin promoter requires binding of the transcription factor CBFA1, also known as Runx2, to a response element, named OSE2, in the osteocalcin promoter.

Runx factors are DNA binding proteins that can facilitate tissue-specific gene activation or repression (Lutterbach, B., and S. W. Hiebert. (2000) Gene 245:223-235 ). Mammalian Runx-related genes are essential for blood, skeletal, and gastric development and are commonly mutated in acute leukemias and gastric cancers (Lund, A. H., and M. van Lohuizen. (2002) Cancer Cell. 1:213-215). Runx factors exhibit a tissue-restricted pattern of expression and are required for definitive hematopoiesis and osteoblast maturation. Runx proteins have recently been shown to interact through their C-terminal segment with Smads, a family of signaling proteins that regulate a diverse array of developmental and biological processes in response to transforming growth factor (TGF)-β/bone morphogenetic protein (BMP) family of growth factors. Moreover, subnuclear distribution of Runx proteins is mediated by the nuclear matrix-targeting signal, a protein motif present in the C terminus of Runx factors. Importantly, in vivo osteogenesis requires the C terminus of Runx2 containing the overlapping subnuclear targeting signal and the Smad interacting domain. The Runx and Smad proteins are jointly involved in the regulation of phenotypic gene expression and lineage commitment. Gene ablation studies have revealed that both Runx proteins and Smads are developmentally involved in hematopoiesis and osteogenesis. Furthermore, Runx2 and the BMP-responsive Smads can induce osteogenesis in mesenchymal pluripotent cells.

"Runx2" is one of three mammalian homologues of the Drosophila transcription factors, Runt and Lozenge (Daga, A., et al.(1996) Genes Dev. 10:1194-1205). Runx2 is also expressed in T lymphocytes and cooperates with oncogenes c-myc, p53, and Pim1 to accelerate T-cell lymphoma development in mice (Blyth, K., et al. (2001) Oncogene 20:295-302).

Runx2 expression also plays a key role in osteoblast differentiation and skeletal formation. In addition to osteocalcin, Runx2 regulates expression of several other genes that are activated during osteoblast differentiation, including alkaline phosphatase, collagen, osteopontin, and osteoprotegerin ligand. These genes also contain Runx2 - binding sites in their promoters. These observations suggest that Runx2 is an essential transcription factor for osteoblast differentiation. This hypothesis is strongly supported by the absence of bone formation in mouse embryos in which the cbfa1 gene was inactivated. Furthermore, cleidocranial dysplasia, a human disorder in which some bones are not fully developed, has been associated with mutations in a cbfa1 allele. In addition to its role in osteoblast differentiation, Runx2 has been implicated in the regulation of bone matrix deposition by differentiated osteoblasts. The expression of Runx2 is regulated by factors that influence osteoblast differentiation. Accordingly, BMPs can activate, while Smad2 and glucocorticoids can inhibit, Runx2 expression. In addition, Runx2 can bind to an OSE2 element in its own promoter, suggesting the existence of an autoregulatory feedback mechanism of transcriptional regulation during osteoblast differentiation. For a review, see, Alliston, et al. (2000) EMBO J 20:2254.

As described herein, Runx2 interacts with Shn3 through its Runt DNA binding domain. The best-described binding partner for the Runt domain of Runx2 is CBFβ, a constitutively-expressed factor required for high-affinity DNA binding by Runx2 (Tang, Y. Y., et al. (2000). J Biol Chem 275, 39579-39588; Yoshida, C. A., et al. (2002). Nat Genet 32, 633-638). Although CBFβ-/- mice die at E12.5 due to severe defects in Runx1-mediated hematopoiesis, when CBFβ-/- mice are rescued by transgenic overexpression of CBFβ by the Gata1 promoter, severe dwarfism results that mimicking the phenotype of Runx2-/- mice (Yoshida, C. A., et al. (2002). Nat Genet 32, 633-638). When bound to CBFβ, Runx family members are protected from ubiquitin/proteasome-mediated degradation (Huang, G., et al. (2001). Embo J 20, 723-733). When bound to CBFβ, Runx2 stability is promoted and it optimally binds target DNA sequences. When bound to Shn3, Runx2 can no longer bind target sequences with high affinity, and Runx2 degradation is accelerated due to enhanced ubiquitination and subsequent proteolysis.

The nucleotide sequence and amino acid sequence of human Runx2, is described in, for example, GenBank Accession No. gi:10863884. The nucleotide sequence and amino acid sequence of murine Runx2, is described in, for example, GenBank Accession No. gi:20806529. The nucleotide sequence and amino acid sequence of human CBFβ, is described in, for example, GenBank Accession No. gi: 47132615 and 47132616. The nucleotide sequence and amino acid sequence of murine CBFβ, is described in, for example, GenBank Accession No. gi: gi:31981853.

As used herein, "WWP1" is a member of the family of E3 ubiquitin ligases with multiple WW domains, which also includes Nedd4, WWP2, and AIP4. WWP1 has previously been shown to interact with all R- and I-Smad proteins, and to promote the ubiquitination of Smad6 and Smad7 (Komuro, A., et al. (2004). Oncogene 23, 6914-6923); however, the ability of WWP1 to ubiquitinate Runx proteins, which also possess PPXY motifs in their Runt domains (Jin, Y. H., et al. (2004). J Biol Chem 279, 29409-29417), had not been investigated.

The nucleotide sequence and amino acid sequence of human WWP1, is described in, for example, GenBank Accession No. gi:33946331. The nucleotide sequence and amino acid sequence of murine WWP1, is described in, for example, GenBank Accession No. gi:51709071.

"Bone sialoprotein" or "BSP" belongs to the osteopontin gene family and is a non-collagenous bone matrix protein that binds tightly to hydroxyapatite, forming an integral part of the mineralized matrix of bone. The nucleotide sequence and amino acid sequence of human BSP, is described in, for example, GenBank Accession No. gi:38146097. The nucleotide sequence and amino acid sequence of murine BSP, is described in, for example, GenBank Accession No. gi:6678112.

Type I collagen (α)1 ("ColI(α)1"), is a collagenous bone matrix protein. The nucleotide sequence and amino acid sequence of human ColI(α)1, is described in, for example, GenBank Accession No. gi:14719826. The nucleotide sequence and amino acid sequence of murine ColI(α)1, is described in, for example, GenBank Accession No. gi:34328107.

"ATF4", also called "CREB2", and "Osterix", also called "SP7", are transcription factors belonging to the bZIP protein family and C2H2-type zinc-finger protein family, respectively, that are key regulators of bone matrix biosynthesis during remodeling of bone, *e.g*., during bone formation and mineralization (see, for example, Yang, X., et al. (2004). Cell 117, 387-398; Nakashima, K., et al. (2002). Cell 108, 17-2). BSP, ColI(α)1, ATF4, and Osterix are specific markers of bone formation and development. The nucleotide sequence and amino acid sequence of human ATF4, is described in, for example, GenBank Accession No. gi:33469975 and gi:33469973. The nucleotide sequence and amino acid sequence of murine ATF4, is described in, for example, GenBank Accession No. gi:6753127. The nucleotide sequence and amino acid sequence of human SP7, is described in, for example, GenBank Accession No. gi:22902135. The nucleotide sequence and amino acid sequence of murine SP7, is described in, for example, GenBank Accession No gi:18485517.

The term "ATF4 signaling pathway" refers to any one of the signaling pathways known in the art which involve Activating Transcription Factor 4 to regulate osteoblast development and function. As discussed above, ATF4 is a transcription factor which functions as a specific repressor of CRE-dependent transcription. The transcriptional repressor activity resides within the C-terminal leucine zipper and basic domain region of the ATF4 protein. ATF4 has been shown to be required for high levels of collagen synthesis by mature osteoblasts and requires phosphorylation by the kinase, RSK2, for optimal extracellular matrix production by osteoblasts (Yang, et al. (2004) Cell 117:387). Furthermore, as described herein, animals deficient in Shn3 have elevated levels of ATF4 and RSK2 mRNA and protein, as well as an accumulation of hyperphosphorylated ATF4. The nucleotide sequence and amino acid sequence of human RSK2, is described in, for example, GenBank Accession No. gi:56243494. The nucleotide sequence and amino acid sequence of murine Rsk2, is described in, for example, GenBank Accession No. gi:22507356.

The term "AP-1" refers to the transcription factor activator protein 1 (AP-1) which is a family of DNA-binding factors that are composed of dimers of two proteins that bind to one another via a leucine zipper motif. The best characterized AP-1 factor comprises the proteins Fos and Jun. (Angel, P. and Karin, M. (1991) Biochim. Biophys Acta 1072:129-157; Orengo, I. F. , Black, H. S. , et al. (1989) Photochem. Photobiol. 49:71-77; Curran, T. and Franza, B. R., Jr. (1988) Cell 55, 395-397). The AP-1 dimers bind to and transactivate promoter regions on DNA that contain cis-acting phorbol 12-tetradecanoate 13-acetate (TPA) response elements to induce transcription of genes involved in cell proliferation, metastasis, and cellular metabolism (Angel, P. , et al. (1987) Cell 49, 729-739. AP-1 is induced by a variety of stimuli and is implicated in the development of cancer and autoimmune disease. The nucleotide sequence and amino acid sequence of human AP-1, is described in, for example, GenBank Accession No. gi:20127489.

As used herein, the term "TGFβ signaling pathway" refers to any one of the signaling pathways known in the art which involve transforming growth factor beta. A TGFβ signaling pathway is initiated when this molecule binds to and induces a heterodimeric cell-surface complex consisting of type I (TβRI) and type II (TβRII) serine/threonine kinase receptors. This heterodimeric receptor then propagates the signal through phosphorylation of downstream target SMAD proteins. There are three functional classes of SMAD protein, receptor-regulated SMADs (R-SMADs), e.g., SMAD2 and SMAD3, Co-mediator SMADs (Co-SMADs) and inhibitory SMADs (I-SMADs). Following phosphorylation by the heterodimeric receptor complex, the R-SMADs complex with the Co-SMAD and translocate to the nucleus, where in conjunction with other nuclear proteins, they regulate the transcription of target genes (Derynck, R., et al. (1998) Cell 95: 737-740).

The nucleotide sequence and amino acid sequence of human SMAD2, is described in, for example, GenBank Accession No. gi:20127489. The nucleotide sequence and amino acid sequence of murine SMAD2, is described in, for example, GenBank Accession No. gi:31560567. The nucleotide sequence and amino acid sequence of human SMAD3, is described in, for example, GenBank Accession No. gi:42476202. The nucleotide sequence and amino acid sequence of murine SMAD3, is described in, for example, GenBank Accession No. gi:31543221.

The language "disorders that would benefit from the modulation of Shn3 activity or expression" or "Shn3 associated disorder" includes disorders in which Shn3 activity is aberrant or which would benefit from modulation of a Shn3 activity. Exemplary Shn3 associated disorders include disorders, diseases, conditions or injuries in which modulation of bone formation and mineralization would be beneficial.

### 2. Methods of the Invention

The invention pertains, at least in part, to a method for treating a bone mass disorder. The method includes administering to a subject an effective amount of a Shn3 modulating compound.

The term "Shn3 modulating compound" refers to a compound capable of modulating a Shn3 biological activity such that bone formation and mineralization is modulated, e.g., increased or decreased. In a preferred embodiment, the term "compound" does not include nucleic acid molecules, antisense, siRNA molecules, or dominant negative forms of molecules in the Shn3 osteoblast pathway. Examples of portions of a Shn3 biological activity that may be modulated include the association of Shn3 with WWP1, the association of Shn3/WWP1 with Runx2, the ubiquination of Runx2, or the ability of Runx2 to participate in the transcription of genes involved in the extracellular matrix biosynthesis. In one embodiment, the compound increases osteoblast activity. In another embodiment, the compound decreases osteoblast activity. In a further embodiment, the compound inhibits the Shn3 and WWP1 association. The compound may bind to a biomolecule which results in a Shn3 biological activity being modulated. For example, the compound may bind to WWP1, Shn3, SMAD3, and/or Runx2.

In an embodiment, the Shn3 modulating compound for the methods and pharmaceutical compositions of the invention is 5,5'-(sulfonyldimethylene)diuracil.

The Shn3 modulating compound may increase osteoblast activity by about 1 % or more, about 5% or more, about 10% or more, about 15% or more, about 20% or more, about 25% or more, about 30% or more, about 35% or more, about 40% or more, about 45% or more, about 50% or more, about 55% or more, about 60% or more, about 65% or more, about 70% or more, about 75% or more, about 80% or more, about 85% or more, about 90% or more, about 95% or more, or about 100% or more.

Modulation of osteoblast activity can be measured *in vitro* or *in vivo.* For example, various *in vitro* techniques for determining the ability of compound to modulate bone formation and mineralization are known to the skilled artisan. For example, skeletal architecture can be assayed by digital radiography of, trabeculation (i.e., the anastomosing bony spicules in cancellous bone which form a meshwork of intercommunicating spaces that are filled with bone marrow) can be determined by three-dimensional µ-QCT imaging, and by analyses of bone cross-sections. In addition, trabecular number, trabecular thickness, bone volume per tissue volume (BV/TV), and bone mineral density (BMD) can also be determined by µ-QCT imaging. These analyses can be performed on whole skeleton preparations or individual bones. Mineralized bone and non-mineralized cartilage formation can be determined by histochemical analyses, such as by alizarin red/alcian blue staining. To assay a compound for an effect on osteoblast function versus osteoclast function, *in vitro* osteoclast differentiation assays are performed by culturing bone marrow (BM) in the presence of M-CSF and RANKL to generate TRAP+ osteoclasts. *In vivo* determinations of whether a compound effects osteoblast function or osteoclast can be performed by, for example, bone marrow transfers. In addition, various histomorphometric parameters can be analyzed to determine bone formation rates. For example, dual calcein-labeling of bone visualized with fluorescent micrography allows the determination of bone formation rate (BFR), which is calculated by multiplying the mineral apposition rate (MAR), which is a reflection of the bone formation capabilities of osteoblasts, by the area of mineralized surface per bone surface (MS/BS). In addition, the total osteoblast surface, which a reliable indicator of osteoblast population, can be measured, as can osteoid thickness, i.e., the thickness of bone that has not undergone calcification. Sections of bone can also be analyzed by staining with Von Kossa and Toluidine Blue for analysis of *in vivo* bone formation. The *ex vivo* culturing of osteoblast precursors and immature osteoblasts can also be performed to determine if cells possess the capacity to form mineralized nodules, which reflects the generation of extracellular matrix, i.e., the mineralized matrix of bone. Furthermore, these cultures can be assayed for their proliferative ability, *e.g*., by cell counting, and can be stained for the presence of various markers of bone formation, such as for example, alkaline phosphatase. These same cultures can also be used for various analyses of mRNA and protein production of numerous molecules known to be involved in bone formation and mineralization, and osteoclastogenesis, such as, for example, BSP, ColI(α)1, and OCN, ALP, LRP5, Osterix, Runx2, RANKL, and ATF4.

Examples of disorders in which inhibition of Shn3 activity is desirable include those situations in which Shn3 is abnormally upregulated and/or in which decreased Shn3 activity is likely to have a beneficial effect. Increasing bone formation and mineralization by inhibiting Shn3 activity is useful in situations in which increased bone formation and mineralization would be beneficial. For example, osteoporosis, including idiopathic osteoporosis, secondary osteoporosis, transient osteoporosis of the hip, osteomalacia, skeletal changes of hyperparathyroidism, chronic renal failure (renal osteodystrophy), osteitis deformans (Paget's disease of bone), osteolytic metastases, and osteopenia in which there is progressive loss of bone density and thinning of bone tissue are conditions which would benefit from increased bone formation and mineralization such that breaks and/or fractures would not occur. Osteoporosis and osteopenia can result not only from aging and reproductive status, but can also be secondary to numerous diseases and disorders, as well as due to prolonged use of numerous medications, *e.g*., anticonvulsants (*e.g*., for epilepsy), corticosteroids (e.g., for rheumatoid arthritis and asthma), and/or immunosuppressive agents (e.g., for cancer). For example, glucocorticoid-induced osteoporosis is a form of osteoporosis that is caused by taking glucocorticoid medications such as prednisone (Dehasone, Orasone, etc.), prednisolone (Prelone), dexamethasone (Decadron, Hexadrol), and cortisone (Cortone Acetate). These medications are frequently used to help control many rheumatic diseases, including rheumatoid arthritis, systemic lupus erythematosus, inflammatory bowel disease, and polymyalgia rheumatica. Other diseases in which osteoporosis may be secondary include, but are not limited to, juvenile rheumatoid arthritis, diabetes, osteogenesis imperfecta, hyperthyroidism, hyperparathyroidism, Cushing's syndrome, malabsorption syndromes, anorexia nervosa and/or kidney disease. In addition, numerous behaviors have been associated with osteoporosis, such as, prolonged inactivity or immobility, inadequate nutrition (especially calcium, vitamin D), excessive exercise leading to amenorrhea (absence of periods), smoking, and/or alcohol abuse. Furthermore, promoting the induction of bone formation and mineralization may be beneficial to treat, for example a bone fracture or break, a tooth replacement, either replacement of a subjects' own tooth or a prosthetic tooth, or ameliorate symptoms of an ongoing condition, such as for example, bone loss associated with, for example perimenopause or menopause. In addition, compounds of the invention which stimulate Shn3 activity as a means of downmodulating bone formation and mineralization is also useful in therapy. For example, decreasing or inhibiting bone formation and mineralization by enhancing Shn3 is beneficial in diseases, disorders, conditions or injuries in which there is premature fusing of two or more bone, or bone density is too high, such as for example, craniosynostosis (synostosis), osteopetrosis (including malignant infantile form, intermediate form, and adult form), primary extra-skeletal bone formation, *e.g*., multiple miliary osteoma cutis of the face, and osteitis condensans.

The term "subjects" includes organisms with bones. In a further embodiment, the subject is a mammal, *e.g*., a rat, mouse, rabbit, goat, horse, sheep, dog, cat, pig, cow, bear, monkey, gorilla, ferret, guinea pig, or, preferably, a human. The subject may have or be at risk of having a bone disorder, such as described above. In another further embodiment, the subject is over 40 years of age, over 50 years of age, over 60 years of age, over 65 year of age, over 70 years of age, over 75 years of age, over 80 years of age, over 85 years of age, over 90 years of age, or over 95 years of age. In another embodiment, the subject is postmenopausal. In another embodiment, the subject is female. In yet another embodiment, the subject has had an ovariectomy or hysterectomy.

The term "treated," "treating" or "treatment" includes therapeutic and/or prophylactic treatment. The treatment includes the diminishment or alleviation of at least one symptom associated or caused by the bone mass disorder. For example, treatment can be diminishment of one or several symptoms of a disorder or complete eradication of the bone disorder as described herein.

### 3. Compounds of the Invention

The invention also pertains, at least in part, to a compound useful for the modulation of bone formation and mineralization and/or Shn3 activity.

The compound of the invention may be synthesized using methods such as those described in Giner-Sorolla et al., J. Med Chem. (1966), 9(1), 97-101 or Giner-Sorolla et al., Nucleic Acid Chem. (1978), 1, 83-87.

In certain embodiments of the invention, the compound of the invention may meet at least one requirement of Lipinski's Rule of Five for an orally bioavailable drug. For example, the compound of the invention may have no more than five hydrogen bond donors (e.g., NH, OH, etc.), no more than ten hydrogen bond acceptors (N, O, etc.), a molecular weight under 500, and/or a partition coefficient of log *P* under 5. In a further embodiment, the compound may also meet one or more requirement of Ghose's rules. Examples of these rules include: a partition coefficient log *P* of between about -0.4 to about +5.6; a molar refractivity of about 40 to about 130; a molecular weight of about 160 to about 480; and about 20 to 70 heavy atoms.

The term "partition coefficient" is a measure of differential solubility of a compound in two solvents. The logarithmic ratio of the concentrations of the solute in the solvent is called log *P* (sometimes LogP). The best known of these partition coefficients is the one based on the solvents octanol and water. The octanol-water partition coefficient is a measure of the hydrophobicity and hydrophilicity of a substance. The classical method of log *P* determination is the shake-flask method, which consists of mixing a known amount of solute in a known volume of octanol and water, then measuring the distribution of the solute in each solvent. The most common method of measuring the distribution of the solute is by UV/VIS spectroscopy.

The term "molar refractivity" is a measure of the volume occupied by an atom or group and is dependent on the temperature, the index of refraction, and the pressure.

As set out above, certain embodiments of the present compound can contain a basic functional group, such as amino or alkylamino, and are, thus, capable of forming pharmaceutically acceptable salts with pharmaceutically acceptable acids. The term "pharmaceutically acceptable salts" is art recognized and includes relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, *e.g*., Berge et al. (1977) "Pharmaceutical Salts", J. Farm. SCI. 66:1-19).

In other cases, the compound of the present invention may contain one or more acidic functional groups and, thus, are capable of forming pharmaceutically acceptable salts with pharmaceutically acceptable bases. The term "pharmaceutically acceptable salts" in these instances includes relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

### 4. Pharmaceutical Compositions

The invention also pertains at least in part to pharmaceutical compositions for the modulation of bone formation or mineralization, treatment of a Shn3 associated disorder, or other disorder treatable by administration of compounds of the invention. The pharmaceutical compositions comprise a compound of the invention in combination with a pharmaceutical acceptable carrier. The composition may further comprise a second agent for the treatment of a bone mass disorder.

In certain embodiments of the invention, the compound is capable of being administered orally to a subject such that said subject's bone mineralization or formation is modulated.

The language "pharmaceutical composition" includes preparations suitable for administration to mammals, e.g., humans. When the compound of the present invention is administered as a pharmaceutical to mammals, *e.g*., humans, it can be given per se or as a pharmaceutical composition containing, for example, 0.1 to 99.5% (more preferably, 0.5 to 90%) of active ingredient in combination with a pharmaceutically acceptable carrier.

The phrase "pharmaceutically acceptable carrier" is art recognized and includes a pharmaceutically acceptable material, composition or vehicle, suitable for administering compounds of the present invention to mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, α-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical, transdermal, buccal, sublingual, rectal, vaginal, pulmonary and/or parenteral administration. In addition, formulation of the present invention may be suitable for administration to cells in ex vivo treatment protocols, or delivered on a surface, *e.g*., a biocompatible surface, for example on the surface of a surgically implanted device, *e.g*., as, for example, a putty, for the stabilization, replacement, etc., of a bone, joint, tooth, etc. The formulations may conveniently be presented in unit dosage form and may be prepared by any methods well known in the art of pharmacy. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert dilutents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane. Sprays also can be delivered by mechanical, electrical, or by other methods known in the art.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or geL

Ophthalmic formulations, eye ointments, powders, solutions and the like, are also contemplated as being within the scope of this invention.

Pharmaceutical compositions of this invention suitable for parenteral administration comprise the compound of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial, antiparasitic and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form may be accomplished by dissolving or suspending the drug in an oil vehicle. The compositions also may be formulated such that its elimination is retarded by methods known in the art.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

The preparations of the present invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration or administration via inhalation is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually. Other methods for administration include via inhalation.

The compound of the invention may also be administered to a subject via stents. The compound may be administered through the stent or be impregnated in the stent itself.

The compound of the invention may also be administered on a surface, *in vitro* or *in vivo.* For example, the surface of a surgically implanted, rod, pin, plate, screw, or other implement implanted for the purpose of stabilizing, repairing a bone, *e.g*., a fracture, a joint, a tooth, or a joint replacement, or a tooth replacement,

Regardless of the route of administration selected, the compound of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, intravenous and subcutaneous doses of the compounds of this invention for a patient will range from about 0.0001 to about 100 mg per kilogram of body weight per day, more preferably from about 0.01 to about 50 mg per kg per day, and still more preferably from about 1.0 to about 100 mg per kg per day.

If desired, the effective daily dose of the active compound may be administered as two, three, four, five, six or more sub-doses administered separately at appropriate intervals throughout the day, optionally, in unit dosage forms.

While it is possible for the compound of the present invention to be administered alone, it is preferable to administer the compound as a pharmaceutical composition. Compounds or pharmaceutical compositions can be administered in combination with other agents and/or methods. For example, surgical repair, surgical implantation of biodegradable devices, rosiglitazone, RANKL, tretinoin, enoxaparin can be used in conjunction with a compound that decreases bone formation and mineralization. Agents and/or methods suitable for administration in combination with a compound that increases bone formation and mineralization, include, for example, surgery, OP-1^{R}, also known as BMP-7, a member of the Bone Morphogenetic Protein superfamily, BMP-2, vitamin D, calcium, hormone replacement therapy, bisphosphonates, *e.g*., analogues of endogenous pyrophosphates which inhibit bone resorption, such as, for example, alendronate, etidronate, pamidronate, Calcitonin, Clodronate, selective estrogen receptor modulators (SERMs), *e.g*., raloxifene, parathyroid hormone, *e.g*., teriparatide, fluoride, strontium ranelate, TNF-alpha antibodies, osteoprotegerin, beta-Cryptoxanthin, and thiazides can decrease urinary calcium excretion and slow bone loss, tyrosine phosphatase inhibitors, *e.g*., sodium orthovanadate, alfacalcidol, menatetrenone, statins, *e.g*., simvastatin.

As set out above, certain embodiments of the present compounds can contain a basic functional group, such as amino or alkylamino, and are, thus, capable of forming pharmaceutically acceptable salts with pharmaceutically acceptable acids. The term "pharmaceutically acceptable salts" is art recognized and includes relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, *e.g*., Berge et al. (1977) "Pharmaceutical Salts", J. Farm. SCI. 66:1-19).

In other cases, the compound of the present invention may contain one or more acidic functional groups and, thus, are capable of forming pharmaceutically acceptable salts with pharmaceutically acceptable bases. The term "pharmaceutically acceptable salts" in these instances includes relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

The term "pharmaceutically acceptable esters" refers to the relatively non-toxic, esterified products of the compound of the present invention. These esters can be prepared *in situ* during the final isolation and purification of the compounds, or by separately reacting the purified compound in its free acid form or hydroxyl with a suitable esterifying agent. Carboxylic acids can be converted into esters via treatment with an alcohol in the presence of a catalyst. Hydroxyls can be converted into esters via treatment with an esterifying agent such as alkanoyl halides. The term also includes lower hydrocarbon groups capable of being solvated under physiological conditions, *e.g*., alkyl esters, methyl, ethyl and propyl esters. (See, for example, Berge et al., *supra*.)

The invention also pertains, at least in part, to packaged compositions comprising a compound of the invention and instructions for using said compound for the treatment of a bone mass disorder.

The invention is further illustrated by the following examples, which should not be construed as further limiting.

### Exemplification of the Invention:

### Example 1: Schnurri-3 (Shn3) and Osteogenesis.

Shn3 is a potent and essential regulator of adult bone formation. Mice lacking Shn3 display an osteosclerotic phenotype with profoundly increased bone mass due to augmented osteoblast activity. Shn3 controls protein levels of Runx2, the principal regulator of osteoblast differentiation, by promoting its degradation. In osteoblasts, Shn3 functions as a component of a trimeric complex between Runx2 and the E3 ubiquitin ligase WWP1. This complex inhibits Runx2 function and expression of genes involved in extracellular matrix mineralization due to the ability of WWP1 to promote Runx 2 polyubiquitination and proteasome-dependent degradation. Compounds that inhibit WWP1 should elevate osteoblast synthetic activity and hence bone mass.

Histologic and radiographic analysis of femurs from Shn3 mice reveal dramatically increased bone mass and density with obliteration of the marrow cavity.

Reduction of WWPI protein in primary calvarial osteoblasts results in increased levels ofRunx 2 protein, increased levels of bone synthetic genes and increased formation of mineralized bone.

A cell-based reporter assay was modified for use as a primary screen. It is based on the inhibition by WWPI of Runx2 activation of a target promoter sequence from the osteocalcin gene. The murine mesenchymal stem cell line, C3H10T1/2 was maintained in DMEM + 10%FBS. Cells were seeded overnight in a 12-wlel dish at 8x104 cells/well and transfected with the multimerized osteocalcin (OSE) luciferase (6xOSE2) reporter gene plasmid and combinations of expression constructs, as indicated, by Effectene (Qiagen). Total amounts of transfected DNA were kept constant by supplementing with control empty expression vector plasmids. All cells were cotransfected with pRL-TK (Promega) to control for transfection efficience. Forty-eight hours after transfection, cells were harvested and lysed in 1xPassive Lysis Buffer (Promega) and luciferase assays performed using the Dual-Luciferase Reporter Assay System (Promega). Runx2 robustly transactivated the OSE2 reporter and this was substantially inhibited by Shn3 alone, by WWP1 alone and further inhibited by Shn3 and WWP1 together.

To demonstrate that inhibition of WWP1 may result in increased Runx2 activity, C3H10T1/2 cells were infected with GFPi or WWPli lentiviruses. Runx2 tranaactivation function in luciferase reporter assays was enhanced in WWP1i cells. The LKO.1 lentiviral vectors expressing RNAi against murine WWP1, and GFP were cotransfected along with D8.9 and VSV-G plasmids into C2H10T1/2 cells utilizing Effectene (Qiagen).

5,5'-(sulfonyldimethylene)diuracil was tested in the OSE2 reporter assay and shown to increase Runx2 transactivation, and therefore also inhibit WWP1.

### Example 2: Human Osteoblast Differentiation in Vitro

This cell-based assay uses a 96-well format in which primary human mesenchymal stem cells (MSCs) are differentiated into osteoblasts following an established protocol that results in a high rate of differentiation. To induce osteoblast differentiation, MSC are seeded at a low density (3.1x10³ cells per cm2) and cultured in media containing β-glycerolphosphate and ascorbic acid for fourteen days. For testing of candidate compounds, MSCs will be differentiated in the presence of the compounds for the duration of the 14-day culture period. Osteoblast differentiation is then assayed via a simple colorometric readout that reflects the levels of cellular alkaline phosphates (ALP), an enzyme present in differentiating osteoblasts but absent in MSCs. ALP levels are then normalized to cell number, which is measured by utilizing the Alamar blue assay. Mineralization can be assessed by staining with xylenol orange. The screen and the 96-well format will allow multiple compounds to be tested at various concentrations. A substantial number of compounds may be identified that are active at a nanomolar concentration.

5,5'-(Sulfonyldimethylene)diuracil was identified as a tight binder to WWP1 in the in silico screen for osteoblast differentiation. Inclusion of this compound in the culture system resulted in substantially increased formation of mineralized nodules.

### Example 3: WWP1 Ubiquitination and Runx2 Protein Levels

Once compounds that augment osteoblast differentiation ofMSCs have been identified, it will be determined if these compounds function by antagonizing WWP1 activity *in vitro.* To test this, an *in vitro* ubiquitination assay using the HECT domain of WWP1 will be used as an E3 ligase. Recombinant HECT domain, which contains the catalytic domain of WWP1 is added to the reaction along with ubiquitin and biotinylated ubiquitin with or without recombinant E1, and E2 (UbcH7) along with increasing concentrations of the candidate compounds. Ubiquitination reactions are allowed to proceed at 30°C for 15 minutes, and reactions are resolved by SDS-PAGE, transferred to PVDF membranes, and ubiquitinated proteins are visualized by blotting with streptavidin-HRP. Overall levels of protein ubiquitination (predominantly WWP1 auto-ubiquitination in this assay) are quantified by densitometry in the presence of absence of inhibitors.

It was found that 5,5'-(sulfonyldimethylene)diuracil had an inhibitory effect on WWP1 ubiquitination.

In addition, it will be determined whether or not inhibitors block WWP1-mediated ubiquitination of Runx2 using a cell based system. A 293T cell-based system will be used and to this system increasing amounts of lead compounds are added to the cells 18 hours prior to lysis. Finally, to determine if potential WWP1 inhibitors can block the function of endogenous WWP1 in osteoblasts, a hMSCs will be treated as above with inhibitors during osteoblast differentiation and the Runx2 mRNA and protein levels will be analyzed as described above.

### Example 4: Optimization of Lead Compounds and In Vivo Animal Screening.

Once compounds that enhance *in vitro* osteoblast differentiation through antagonizing WWP1 have been identified, the chemistry of the lead candidates are optimized. The best candidate molecules from laboratory testing may be subjected to rounds of *in silico* analog selection from other chemical libraries or using synthetic chemistry techniques.

The efficacy of some compounds in preventing bone loss *in vivo* is studied. Dose response curves are generated to establish the optimal dose for *in vivo* use. The ability of the compounds to prevent the onset of osteopenia in mice following ovariectomy is tested. Similar to postmenopausal women, estrogen levels decline sharply in mice following ovariectomy. In these experiments, there are two groups of 8 female mice (12 weeks of age) with one group receiving ovariectomy surgery and the other group receiving sham surgery. Mice within each group are administered either the candidate compound or vehicle prior to surgery. The mice continue to receive the candidate compounds or vehicle at various time points post surgery.

Eight-weeks after surgery, µ-QCT analysis is performed on the femur and vertebrae of each mouse to quantitate bone loss by measuring trabecular number, thickness, and spacing, bone volume, and volumetric cone mineral density. Serum is collected prior to sacrifice to measure circulating levels of Trap5b and deoxypyridinoline (Dpd). Uteri of the mice are also be excised and weighed to evaluate the effects of ovariectomy. To determine if the candidate compounds specifically target WWP1 *in vivo,* a transgenic mouse strain will be used that overexpresses human WWP1 (hWWP1) specifically in osteoblasts.

## Claims

1. An Shn3 modulating compound for use in a method of increasing bone formation and mineralization, wherein said compound is: 5,5'-(sulfonyldimethylene)diuracil.

2. The compound for use as claimed in claim 1, wherein said increasing bone formation and mineralization comprises elevated osteoblast synthetic activity, elevated bone mass, increased bone density, increased level of bone synthetic genes, or increased formation of mineralized bone.

3. The compound for use as defined in claim 1, wherein said use is for treating osteoporosis, idiopathic osteoporosis, secondary osteoporosis, transient osteoporosis of the hip, osteomalacia, skeletal changes of hyperparathyroidism, chronic renal failure (renal osteodystrophy), osteitis deformans (Paget's disease of bone), osteolytic metastases or osteopenia.

4. The compound for use as claimed in claim 3, wherein said use is for treating osteoporosis.

5. The compound for use as claimed in claim 1, wherein administration of said compound to a subject is oral, nasal, topical, transdermal, buccal, sublingual, rectal, vaginal, pulmonary and/or parenteral.

6. A pharmaceutical composition, comprising a pharmaceutically acceptable carrier and an effective amount of the compound as defined in claim 1.

7. A pharmaceutical composition comprising
an orally effective amount of a compound of claim 1 for increasing bone formation and mineralization, comprising elevated osteoblast synthetic activity, elevated bone mass, increased bone density, increased level of bone synthetic genes, or increased formation of mineralized bone, and
a pharmaceutically acceptable carrier.

## Patentansprüche

1. Shn3-modulierende Verbindung zur Verwendung in einem Verfahren zur Steigerung der Knochenbildung und -mineralisierung, wobei die Verbindung 5,5'-(Sulfonyldimethylen)diuracil ist.

2. Verbindung zur Verwendung gemäß Anspruch 1, wobei die Steigerung der Knochenbildung und -mineralisierung umfasst erhöhte Osteoblast-synthetische Aktivität, erhöhte Knochenmasse, gesteigerte Knochendichte, gesteigerter Spiegel an Knochen synthetisierenden Genen oder gesteigerte Bildung mineralisierten Knochens.

3. Verbindung zur Verwendung gemäß Anspruch 1, wobei die Verwendung für Behandlung von Osteoporose, idiopathischer Osteoporose, sekundärer Osteoporose, transienter Osteoporose der Hüfte, Osteomalazie, skelettalen Veränderungen von Hyperparathyreoidismus, chronischem Nierenversagen (renale Osdeodystrophie), Osteodystrophia deformans (Paget-Syndrom des Knochens), osteolytischer Metastasen oder Osteopenie ist.

4. Verbindung zur Verwendung gemäß Anspruch 3, wobei die Verwendung für Behandlung von Osteoporose ist.

5. Verbindung zur Verwendung gemäß Anspruch 1, wobei Verabreichung der Verbindung an einen Patienten oral, nasal, topisch, transdermal, bukkal, sublingual, rektal, vaginal, pulmonal und/oder parenteral ist.

6. Pharmazeutische Zusammensetzung, umfassend einen pharmazeutisch annehmbaren Träger und eine wirksame Menge einer Verbindung aus Anspruch 1.

7. Pharmazeutische Zusammensetzung, umfassend
eine oral wirksame Menge einer Verbindung aus Anspruch 1 zur Steigerung der Knochenbildung und -mineralisierung, umfassend erhöhte Osteoblast-synthetische Aktivität, erhöhte Knochenmasse, gesteigerte Knochendichte, gesteigerter Spiegel an knochensynthetisierenden Genen oder gesteigerte Bildung mineralisierten Knochens, und
einen pharmazeutisch annehmbaren Träger.

## Revendications

1. Un composé modulateur de Shn3 pour une utilisation dans un procédé d'augmentation de la formation et de la minéralisation osseuses, où ledit composé est : 5,5'-(sulfonyldiméthylène)diuracil.

2. Le composé pour une utilisation selon la revendication 1, où lesdites formation et minéralisation osseuses comprennent une activité synthétique des ostéoblastes élevée, une masse osseuse élevée, une densité osseuse accrue, un niveau accru des gènes artificiels de l'os ou une formation accrue d'os minéralisé.

3. Le composé destiné à une utilisation selon la revendication 1 où ladite utilisation est le traitement de : ostéoporose, ostéoporose idiopathique, ostéoporose secondaire, ostéoporose transitoire de la hanche, ostéomalacie, modifications squelettiques de l'hyperparathyroïdisme, insuffisance rénale chrnique (ostéodystrophie rénale), osteitis deformans (maladie de Paget de l'os), métastases ostéolytiques ou ostéopénie.

4. Le composé destiné à une utilisation selon la revendication 3 où ladite utilisation est pour le traitement de l'ostéoporose.

5. Le composé destiné à une utilisation selon la revendication 1 où l'administration dudit composé à un sujet est orale, nasale, topique, transdermique, buccale, sublinguale, rectale, vaginale, pulmonaire et/ou parentérale.

6. Une composition pharmaceutique comprenant un véhicule pharmaceutiquement acceptable et une quantité efficace du composé selon la revendication 1.

7. Une composition pharmaceutique comprenant
une quantité oralement efficace d'un composé selon la revendication 1 destinée à augmenter la formation et la minéralisation osseuses, comprenant une activité synthétique des ostéoblastes élevée, une masse osseuse élevée, une densité osseuse accrue, un niveau accru des gènes artificiels de l'os ou une formation accrue d'os minéralisé, et
un véhicule pharmaceutiquement acceptable.
